# NEW EUROPEAN PATENT SPECIFICATION

(11) **EP 3 187 064 B2**
(45) Date of publication and mention of the opposition decision: **30.07.2025**
(45) Mention of the grant of the patent: 30.09.2020
(21) Application number: 15836905.8
(22) Date of filing: 18.08.2015
(51) Int. Cl.: A44B 18/00, A61F 13/62, B32B 5/26, D04H 1/541, D04H 1/559, D04H 3/147, D04H 11/08

(54) **HOOK-AND-LOOP FASTENER FEMALE MEMBER**
AUFNAHMEELEMENT EINES KLETTVERSCHLUSSES
ÉLÉMENT FEMELLE DE FERMETURE AUTOAGRIPPANTE

(30) Priority: 26.08.2014 JP 2014171497; 17.07.2015 JP 2015143006; 17.08.2015 JP 2015160254
(43) Date of publication of application: 05.07.2017
(73) Proprietor: Nitto Denko Corporation, Ibaraki-shi, Osaka 567-8680 (JP)
(72) Inventor: UCHIDA, Shou, Ibaraki-shi Osaka 567-8680 (JP); IKISHIMA, Shinsuke, Ibaraki-shi Osaka 567-8680 (JP); TAKEDA, Kohei, Ibaraki-shi Osaka 567-8680 (JP); ARAKAWA, Masaaki, Ibaraki-shi Osaka 567-8680 (JP)
(74) Representative: Müller-Boré & Partner Patentanwälte PartG mbB
(86) International application number: PCT/JP2015/073105
(87) International publication number: WO 2016/031623

(56) References cited:
- EP-A1- 0 882 828
- EP-A1- 3 311 688
- EP-A2- 1 986 583
- WO-A1-2008/108238
- WO-A1-2008/130807
- WO-A1-2016/204132
- WO-A1-92/20251
- JP-A- 2011 135 985
- JP-A- 2011 135 985
- JP-A- H10 127 311
- JP-A- H11 335 956
- US-A- 5 786 060
- US-A- 5 891 547
- US-A1- 2006 019 055
- US-A1- 2006 019 572
- US-A1- 2011 118 692

## Description

The present invention relates to a hook-and-loop fastener female member.

Various hook-and-loop fasteners are proposed for materials for articles such as sanitary articles, for example, diapers and masks (see, for example, Patent Literatures 1 and 2). When used for, for example, a diaper, a hook-and-loop fastener female member is generally attached to a front surface of a waist portion of the diaper and allowed to engage with a hook-and-loop fastener male member (typically having an engaging hook) fixed to a side surface of the diaper, to thereby complete a hook-and-loop fastener. The hook-and-loop fastener is required to be able to be removed and attached many times.

In recent years, non-woven fabrics have been adopted for many of the engaging layers of hook-and-loop fastener female members (layers on which the engaging hooks of hook-and-loop fastener male members can engage) to be used for sanitary articles (in particular, disposable diapers, supporters, masks, and the like).

However, the related-art hook-and-loop fastener female member including the engaging layer adopting the non-woven fabric do not have a sufficient engaging force with the hook-and-loop fastener male member. Inparticular, its shearing stress is low or its peeling strength is weak in many cases . Accordingly, in a disposable diaper or the like, there is a problem of slippage, for example, at the time of wearing or after excretion.
[PTL 1] JP 2009-527315 A
[PTL 2] JP 2010-125337 A

The present invention has been made to solve the problem of the related art, and an object of the present invention is to provide a hook-and-loop fastener female member including an engaging layer adopting a non-woven fabric, the hook-and-loop fastener female member being excellent in engaging force with a hook-and-loop fastener male member.

A hook-and-loop fastener female member according to one embodiment of the present invention includes an engaging layer engageable with a male member, and a physical property layer,
in which the engaging layer includes a non-woven fabric of fiber,
in which the non-woven fabric in the engaging layer has a density of from 50 kg/m³ to 100 kg/m³,
in which the fiber has a diameter of from 15 µm to 60 µm,
wherein the engaging layer is laminated on the physical property layer by embossing using a pattern roll, wherein a surface of the fiber forming the non-woven fabric included in the engaging layer and a surface of the physical property layer on an engaging layer side contain the same kind of polymer, wherein the polymer comprises polyolefin, and wherein the hook-and-loop fastener female member has a shearing stress of 8 N to 100 N.

In a preferred embodiment, the fiber includes a polyolefin fiber, and wherein the polyolefin fiber comprises polypropylene fiber.

In a preferred embodiment, the fiber includes a composite fiber of two or more kinds of resins, wherein the composite fiber of two or more kinds of resins includes crimpable fiber having a core-sheath structure, wherein a sheath portion of the core-sheath structure contains a polyethylene resin.

In a preferred embodiment, a core portion of the core-sheath structure contains a polyethylene terephthalate resin.

In an embodiment, which is not according to the invention, the non-woven fabric in the engaging layer has a density of from 8 kg/m³ to 50 kg/m³.

In a preferred embodiment, the non-woven fabric in the engaging layer has a density of from 70 kg/m³ to 100 kg/m³,

In a preferred embodiment, the fiber has a diameter of from 15 µm to 40 µm.

In a preferred embodiment, the fiber has a diameter of from 20 µm to 40 µm.

In a preferred embodiment, the physical property layer includes a non-woven fabric of fiber, and the fiber has a diameter of 40 µm or less.

In a preferred embodiment, the physical property layer includes a film, and the film has a thickness of 60 µm or less.

In a preferred embodiment, the hook-and-loop fastener female member according to the embodiment of the present invention has a shearing stress of 10 N or more.

In a preferred embodiment, the hook-and-loop fastener female member according to the embodiment of the present invention has a shearing stress of 20 N or more.

A hook-and-loop fastener according to one embodiment of the present invention includes : the hook-and- loop fastener female member according to the embodiment of the present invention; and a hook-and-loop fastener male member configured to engage with the hook-and-loop fastener female member.

A sanitary article according to one embodiment of the present invention includes the hook-and-loop fastener female member according to the embodiment of the present invention.

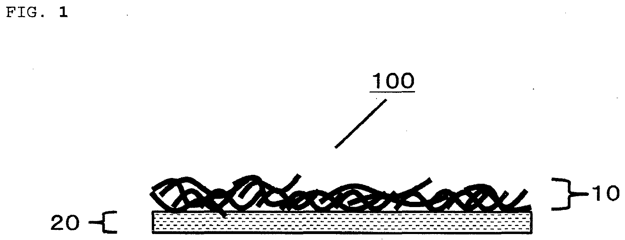

FIG. 1 is a schematic cross-sectional view of a hook-and-loop fastener female member according to a preferred embodiment of the present invention.

### <<Hook-and-loop Fastener Female Member>>

Ahook-and-loop fastener female member of the present invention is a hook-and-loop fastener female member including an engaging layer engageable with a male member (sometimes referred to as "mechanical hook member"). The engaging layer of the hook-and-loop fastener female member is specifically a layer on which an engaging hook (or something having properties equivalent to those of the engaging hook) of a hook-and-loop fastener male member is engageable. A product including the hook-and-loop fastener female member of the present invention and a hook-and-loop fastener male member configured to engage with the hook-and-loop fastener female member serves as a hook-and-loop fastener.

The hook-and-loop fastener female member of the present invention may include any appropriate other member as long as the hook-and-loop fastener female member includes the engaging layer and the physical property layer (layer to be arranged mainly in order to support or reinforce the engaging layer, carry out fixing or adhesion to another article, or form a printed layer on the female member), and the effect of the present invention is not impaired.

The thickness of the hook-and-loop fastener female member of the present invention may be set to any appropriate thickness depending on the purpose. Typically, the thickness of the hook-and-loop fastener female member of the present invention is preferably from 0.2 mm to 5.0 mm, more preferably from 0.3 mm to 4.0 mm, still more preferably from 0.5 mm to 3.0 mm, particularly preferably from 0.5 mm to 2.0 mm. In the present invention, the thickness of the hook-and-loop fastener female member (non-woven fabric) is measured on the basis of a method to be described later.

FIG. 1 is a schematic cross-sectional view of a hook-and-loop fastener female member according to a preferred embodiment of the present invention. In FIG. 1, a hook-and-loop fastener female member 100 includes an engaging layer 10 and a physical property layer 20.

The engaging layer includes a non-woven fabric of fiber. The number of layers of the engaging layer may be only one, or may be two or more. The engaging layer is preferably formed only of the non-woven fabric of fiber.

The number of kinds of the non-woven fabric of fiber included in the engaging layer may be only one, or may be two or more.

Examples of the non-woven fabric of fiber included in the engaging layer include a spunbonded non-woven web, a fluffy non-woven fabric (e.g., a non-woven fabric obtained by a thermal bonding method, a bonding joiningmethod, a spunlace method, or an air-through method), a meltblown non-woven web, a spunlace non-woven web, a spunbonded meltblown spunbonded non-woven web, a spunbonded meltblown meltblown spunbonded non-woven web, an unjoined non-woven web, an electrospun non-woven web, a flashspun non-woven web (e.g., TYVEKTM from DuPont), and a carded non-woven fabric.

The non-woven fabric of fiber included in the engaging layer may contain fiber that is a homogeneous structural body, or may contain a bicomponent structural body, such as a core-sheath structure, a side-by-side structure, a sea-island structure, and any other bicomponent structure. Detailed descriptions of the non-woven fabric may be found in, for example, "Nonwoven Fabric Primer and Reference Sampler," E.A. Vaughn, Association of the Nonwoven Fabrics Industry, third edition (1992).

Any appropriate fiber may be adopted as the fiber forming the non-woven fabric included in the engaging layer as long as the surface of the fiber forming the non-woven fabric included in the engaging layer and the surface of the physical property layer on the engaging layer side contain the same kind of polymer, and the polymer comprising polyolefin. For example, the non-woven fabric may contain fiber of polypropylene, polyethylene, a copolymer thereof, or a blend thereof, or a mixture thereof, or any other polyolefin. Such fiber is preferably at least one kind selected from polyolefin fiber, and composite fiber of two or more kinds of resins because the effect of the present invention can be more expressed.

Examples of the polyolefin fiber include polypropylene fiber, polyethylene fiber, and α-olefin copolymer fiber. The polyolefin fiber is preferably polypropylene fiber because the effect of the present invention can be more expressed.

Examples of the composite fiber of two or more kinds of resins include crimpable fiber that is described later, heat-resistant fiber, and hollow fiber. Examples of the composite fiber of two or more kinds of resins include crimpable fiber having a core-sheath structure, crimpable fiber having a side-by-side structure, and heat-resistant fiber having a core-sheath structure, which are preferred because the effect of the present invention can be more expressed. Specific examples thereof include: crimpable fiber having a core-sheath structure in which its core portion contains a polyester resin (preferably a polyethylene terephthalate (PET) resin) or a polyolefin resin (preferably a polypropylene resin) and its sheath portion contains a polyolefin resin (preferably a polypropylene resin or a polyethylene resin, more preferably a polyethylene resin) ; and crimpable fiber in which a polyester resin (preferably a polyethylene terephthalate (PET) resin) or a polyolefin resin (preferably a polypropylene resin) and a polyolefin resin (preferably a polypropylene resin or a polyethylene resin, more preferably a polyethylene resin) form a side-by-side structure. In the heat-resistant fiber having a core-sheath structure, the case where its sheath portion contains a polyethylene resin is preferred because the surface of the heat-resistant fiber is easily roughened, and hence a hook-and-loop fastener male member is easily hooked. In addition, in the heat-resistant fiber having a core-sheath structure, the case where its core portion contains a polyethylene terephthalate (PET) resin is preferred because the breaking strength of the heat-resistant fiber is increased and the engaging force is increased.

The fiber forming the non-woven fabric included in the engaging layer may contain any appropriate other component as long as the effect of the present invention is not impaired. Examples of such other component include other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. They may be used alone or in combination. The content of the other component in the fiber forming the non-woven fabric included in the engaging layer is preferably 10 wt% or less, more preferably 7 wt% or less, still more preferably 5 wt% or less, particularly preferably 2 wt% or less, most preferably 1 wt% or less.

In the hook-and-loop fastener female member of the present invention, when importance is put on the engaging force, the density of the non-woven fabric in the engaging layer is from 50 kg/m³ to 100 kg/m³, more preferably from 50 kg/m³ to 80 kg/m³, still more preferably from 50 kg/m³ to 70 kg/m³, particularly preferably from 50 kg/m³ to 60 kg/m³. In the hook-and-loop fastener female member of the present invention, when the density of the non-woven fabric in the engaging layer falls within the above-mentioned range, there can be provided a hook-and-loop fastener female member excellent in engaging force with a hook-and-loop fastener male member. In particular, in the hook-and-loop fastener female member of the present invention, when the density of the non-woven fabric in the engaging layer falls within the above-mentioned range, both high shearing stress and high peeling strength can be achieved as the engaging force with a hook-and-loop fastener male member, and hence, in a disposable diaper or the like, the problem of slippage, for example, at the time of wearing or after excretion can be effectively eliminated. In the hook-and-loop fastener female member of the present invention, when the density of the non-woven fabric in the engaging layer is so low as to fall outside the above-mentioned range, there is a fear in that a hook-and-loop fastener male member may be hardly hooked or productivity may be poor, leading to an increased cost. When the density of the non-woven fabric in the engaging layer is so high as to fall outside the above-mentioned range, a state in which the fiber of the non-woven fabric of the hook-and-loop fastener female member is densely packed is established, and hence there is a fear that it may be difficult to insert the engaging portion of a hook-and-loop fastener male member into the hook-and-loop fastener female member and an excellent engaging force cannot be expressed.

In the hook-and-loop fastener female member of the present invention, when importance is put on fluffing, the density of the non-woven fabric in the engaging layer is from 50 kg/m³ to 100 kg/m³, particularly preferably from 70 kg/m³ to 100 kg/m³. In the hook-and-loop fastener female member of the present invention, when the density of the non-woven fabric in the engaging layer falls within the above-mentioned range, there can be provided a hook-and-loop fastener female member capable of effectively suppressing fluffing at the time of peeling after engagement with a hook-and-loop fastener male member. In the hook-and-loop fastener female member of the present invention, when the density of the non-woven fabric in the engaging layer is so low as to fall outside the above-mentioned range, there is a fear in that fluffing at the time of peeling after engagement with a hook-and-loop fastener male member may be liable to occur. When the density of the non-woven fabric in the engaging layer is so high as to fall outside the above-mentioned range, a state in which the fiber of the non-woven fabric of the hook-and-loop fastener female member is densely packed is established, and hence there is a fear that it may be difficult to insert the engaging portion of a hook-and-loop fastener male member into the hook-and-loop fastener female member.

In the present invention, the density (kg/m³) of the non-woven fabric in the engaging layer is a value calculated from the basis weight (X g/m²) of the non-woven fabric and the thickness (Y mm) of the non-woven fabric measured on the basis of a method to be described later. More specifically, the density (kg/m³) of the non-woven fabric in the engaging layer is calculated as X/Y (kg/m³).

In the hook-and-loop fastener female member of the present invention, the diameter of the fiber (hereinafter sometimes referred to simply as "fiber diameter") of the non-woven fabric in the engaging layer is from 15 µm to 60 µm, preferably from 18 µm to 60 µm, more preferably from 18 µm to 50 µm, still more preferably from 18 µm to 40 µm, particularly preferably from 20 µm to 40 µm, most preferably from 30 µm to 40 µm. In the hook-and-loop fastener female member of the present invention, when the diameter of the fiber of the non-woven fabric in the engaging layer falls within the above-mentioned range, there can be provided a hook-and-loop fastener female member excellent in engaging force with a hook-and-loop fastener male member. In particular, in the hook-and-loop fastener female member of the present invention, when the diameter of the fiber of the non-woven fabric in the engaging layer falls within the above-mentioned range, both high shearing stress and high peeling strength can be achieved as the engaging force with a hook-and-loop fastener male member, and hence, in a disposable diaper or the like, the problem of slippage, for example, at the time of wearing or after excretion can be effectively eliminated. In the hook-and- loop fastener female member of the present invention, when the diameter of the fiber of the non-woven fabric in the engaging layer is so small as to fall outside the above-mentioned range, there is a fear in that as the engaging force with a hook-and-loop fastener male member, particularly the shearing stress may lower. In addition, in the hook-and-loop fastener female member of the present invention, when the diameter of the fiber of the non-woven fabric in the engaging layer is so small as to fall outside the above-mentioned range, there is a fear in that fluffing of the surface of the hook-and-loop fastener female member may increase. When the diameter of the fiber of the non-woven fabric in the engaging layer is so large as to fall outside the above-mentioned range, there is a fear in that engagement with a hook-and-loop fastener male member may become difficult or production speed may lower, leading to an increased cost. In the present invention, the diameter of the fiber of the non-woven fabric in the engaging layer (fiber diameter) is measured on the basis of a method to be described later.

In the hook-and-loop fastener female member of the present invention, the basis weight of the non-woven fabric in the engaging layer is preferably from 20 g/m² to 60 g/m², more preferably from 20 g/m² to 50 g/m², still more preferably from 20 g/m² to 40 g/m², particularly preferably from 25 g/m² to 40 g/m², most preferably from 30 g/m² to 40 g/m². In the hook-and-loop fastener female member of the present invention, when the basis weight of the non-woven fabric in the engaging layer falls within the above-mentioned range, there can be provided a hook-and-loop fastener female member more excellent in engaging force with a hook-and-loop fastener male member.

In the hook-and-loop fastener female member of the present invention, the fiber of the non-woven fabric in the engaging layer may be crimpable fiber. An example of the crimpable fiber is fiber containing two components having different freezing points, the fiber having a side-by-side structure or an unevenly distributed core-sheath structure, the fiber expressing fine coiled crimps each having a relatively small radius because the component having the higher freezing point first solidifies and shrinks at the time of a phase change from a molten state to a solid state.

Any appropriate material may be adopted as a material for the physical property layer as long as the surface of the fiber forming the non-woven fabric included in the engaging layer and the surface of the physical property layer on the engaging layer side contain the same kind of polymer, and the polymer comprising polyolefin. Examples of the material for the physical property layer include a non-woven fabric of fiber and a film, which are preferred because the effect of the present invention can be more expressed.

When the material for the physical property layer is a non-woven fabric of fiber, the number of kinds of the non-woven fabric may be only one, or may be two or more.

When the material for the physical property layer is a non-woven fabric of fiber, examples of the non-woven fabric include a spunbonded non-woven web, a fluffy non-woven fabric (such as a non-woven fabric obtained by a thermal bonding method, a bonding joining method, a spunlace method, or an air-through method), a meltblown non-woven web, a spunlace non-woven web, a spunbonded meltblown spunbonded non-woven web, a spunbonded meltblown meltblown spunbonded non-woven web, an unjoined non-woven web, an electrospun non-woven web, a flashspun non-woven web (such as TYVEKTM from DuPont), and a carded non-woven fabric.

When the material for the physical property layer is a non-woven fabric of fiber, the non-woven fabric is preferably a non-woven fabric obtained by a spunbond method, a non-woven fabric obtained by a melt blowing method, or a non-woven fabric obtained by combining the foregoing (spunmelt method).

When the material for the physical property layer is a non-woven fabric of fiber, the non-woven fabric may contain fiber that is a homogeneous structural body, or may contain a bicomponent structural body, such as a core-sheath structure, a side-by-side structure, a sea-island structure, or any other bicomponent structure. Detailed descriptions of the non-woven fabric may be found in, for example, "Nonwoven Fabric Primer and Reference Sampler, "E.A. Vaughn, Association of the Nonwoven Fabrics Industry, third edition (1992).

The non-woven fabric may contain fiber of polypropylene, polyethylene, a copolymer thereof, or a blend thereof, or a mixture thereof, or any other polyolefin. Such fiber is preferably at least one kind selected from polyolefin fiber, and composite fiber of two or more kinds of resins because the effect of the present invention can be more expressed.

Examples of the polyolefin fiber include polypropylene fiber, polyethylene fiber, and α-olefin copolymer fiber. The polyolefin fiber is preferably polypropylene fiber because the effect of the present invention can be more expressed.

When the material for the physical property layer is a film, any appropriate material may be adopted as a material for the film as long as the surface of the fiber forming the non-woven fabric included in the engaging layer and the surface of the physical property layer on the engaging layer side contain the same kind of polymer, and the polymer comprising polyolefin. Examples of such material include anunstretchedpolypropylene film, a stretched polypropylene film, and a polyethylene film each having a thickness of from 10 µm to 60 µm, which are preferred because the effect of the present invention can be more expressed.

When the physical property layer is a non-woven fabric of fiber, its basis weight is preferably from 10 g/m² to 40 g/m², more preferably from 10 g/m² to 30 g/m², still more preferably from 10 g/m² to 25 g/m², particularly preferably from 10 g/m² to 20 g/m². When the basis weight of the non-woven fabric in the physical property layer falls within the above-mentioned range, shrinkage deformation in a width direction hardly occurs during web handling, cost competitiveness is excellent, printability is satisfactory, the see-throughproperty of printing is satisfactory, pressure-sensitive adhesive application is easy, the applied pressure-sensitive adhesive hardly exudes to the engaging surface, and flexibility is satisfactory.

When the physical property layer is a non-woven fabric of fiber, the diameter of the fiber is preferably 40 µm or less, more preferably from 1 µm to 40 µm, still more preferably from 1 µm to 30 µm, particularly preferably from 1 µm to 25 µm, most preferably from 1 µm to 20 µm. When the physical property layer is a non-woven fabric of fiber and the diameter of the fiber falls within the above-mentioned range, shrinkage deformation in a width direction hardly occurs during web handling, cost competitiveness is excellent, printability is satisfactory, the see-through property of printing is satisfactory, pressure-sensitive adhesive application is easy, the applied pressure-sensitive adhesive hardly exudes to the engaging surface, and flexibility is satisfactory. When the physical property layer is a non-woven fabric of fiber and the diameter of the fiber is so large as to fall outside the above-mentioned range, there is a fear that shrinkage deformation in a width direction may be liable to occur during web handling, cost competitiveness may be inferior, printability may be poor, pressure-sensitive adhesive application may be difficult, and the applied pressure-sensitive adhesive may have a risk of exuding to the engaging surface.

When the physical property layer is a non-woven fabric of fiber, a laminate of different kinds of non-woven fabrics of fiber (e.g., a laminate of spunbonded non-woven web/meltblown non-woven web/spunbonded non-woven web) may be adopted. In this case, the above-mentioned "diameter of the fiber" refers to the diameter of the fiber of each of the non-woven fabrics.

When the physical property layer is a film, its thickness is preferably 60 µm or less, more preferably from 10 µm to 50 µm, still more preferably from 10 µm to 40 µm, particularly preferably from 10 µm to 30 µm, most preferably from 15 µm to 25 µm. When the physical property layer is a film and its thickness falls within the above-mentioned range, shrinkage deformation in a width direction hardly occurs during web handling, cost competitiveness is excellent, the see-through property of printing is satisfactory, pressure-sensitive adhesive application is easy, and flexibility is satisfactory. When the physical property layer is a film and its thickness is so large as to fall outside the above-mentioned range, there is a fear that cost competitiveness may be inferior, the see-through property of printing may degrade, and flexibility may degrade.

In the hook-and-loop fastener female member of the present invention, the surface of the fiber forming the non-woven fabric included in the engaging layer and the surf ace of the physical property layer on the engaging layer side contain the same kind of polymer. When the surface of the fiber forming the non-woven fabric included in the engaging layer and the surface of the physical property layer on the engaging layer side contain the same kind of polymer, there can be provided a hook-and-loop fastener female member in which fluffing of the engaging layer after peeling after engagement with a hook-and-loop fastener male member is more effectively suppressed. In this case, the "surface of the fiber forming the non-woven fabric included in the engaging layer" may be any surface of the fiber, and encompasses, for example, a sheath portion in fiber having a core-sheath structure.

When the surface of the fiber forming the non-woven fabric included in the engaging layer and the surface of the physical property layer on the engaging layer side contain the same kind of polymer, any appropriate polymer may be adopted as the polymer as long as the polymer is polyolefin. When the surface of the fiber forming the non-woven fabric included in the engaging layer and the surface of the physical property layer on the engaging layer side contain the same kind of polyolefin, there can be provided a hook-and-loop fastener female member in which fluffing of the engaging layer after peeling after engagement with a hook-and-loop fastener male member is even more effectively suppressed.

The hook-and-loop fastener female member of the present invention can achieve, as the engaging force with a hook-and-loop fastener male member, both high shearing stress and high peeling strength, and hence, in a disposable diaper or the like, can effectively eliminate the problem of slippage, for example, at the time of wearing or after excretion.

The hook-and-loop fastener female member of the present invention has a shearing stress to be described in detail later of 8 N or more, more preferably 10 N or more, still more preferably 15 N or more, particularly preferably 20 N or more, most preferably 24 N or more. The upper limit of the shearing stress is 100 N or less in practice when use in a sanitary article and the like are taken into consideration. When the shearing stress of the hook-and-loop fastener female member of the present invention falls within the above-mentioned range, the hook-and-loop fastener female member of the present invention is excellent in engaging force with a hook-and-loop fastener male member.

The hook-and-loop fastener female member of the present invention has a peeling strength (first time) to be described in detail later of preferably 0.2 N or more, more preferably 0.5 N or more, still more preferably 0.8 N or more, particularly preferably 1. 0 N or more. The upper limit of the peeling strength (first time) is preferably 10 N or less in practice when use in a sanitary article and the like are taken into consideration. When the peeling strength (first time) of the hook-and-loop fastener female member of the present invention falls within the above-mentioned range, the hook-and-loop fastener female member of the present invention is excellent in engaging force with a hook-and-loop fastener male member.

The hook-and-loop fastener female member of the present invention has a peeling strength (fourth time) to be described in detail later of preferably 0.2 N or more, more preferably 0.5 N or more, still more preferably 0.8 N or more, particularly preferably 1.0 N or more. The upper limit of the peeling strength (fourth time) is preferably 10 N or less in practice when use in a sanitary article and the like are taken into consideration. When the peeling strength (fourth time) of the hook-and-loop fastener female member of the present invention falls within the above-mentioned range, the hook-and-loop fastener female member of the present invention is excellent in engaging force with a hook-and-loop fastener male member.

### <<Production Method for Hook-and-loop Fastener Female Member of the Present Invention>>

As one preferred production method for the hook-and-loop fastener female member of the present invention, raw non-woven fabrics are laminated and subjected to embossing treatment with a pattern roll at any appropriate treatment temperature (e. g. , 160°C), any appropriate linear pressure (e. g., 160 N/mm), and any appropriate treatment speed (e.g., 10 m/minute) to provide a desired hook-and-loop fastener female member. For example, the pattern used has an embossing portion having any appropriate width (e.g., 1 mm wide), is a square having any appropriate length on a side (e.g., 10 mm on a side), and is placed so that the flow direction of the non-woven fabric and the side form, for example, an angle of 45°.

### <<Application of Hook-and-loop Fastener Female Member of the Present Invention>>

The hook-and-loop fastener female member of the present invention can be used in any appropriate article in which the effect of the present invention can be effectively utilized. A typical example of such article is a sanitary article. That is, a sanitary article of the present invention includes the hook-and-loop fastener female member of the present invention. Examples of such sanitary article include a diaper (in particular, a disposable diaper), a supporter, and a mask.

### Examples

The present invention is hereinafter specifically described by way of Examples. However, the present invention is by no means limited to these Examples. In Examples and the like, test and evaluation methods are as described below. In addition, "part (s)" means "part (s) by weight" and "%" means "wt%" unless otherwise stated.

### <Shearing Stress>

An obtained hook-and-loop fastener female member was cut to a size of 5 cm×5 cm, and the physical property layer surface of the cut hook-and-loop fastener female member was fixed onto a stainless-steel metal sheet through intermediation of a double-sided tape. Meanwhile, a 25 mm×13 mm male member having a guide made of paper (commercially available mechanical hook member for a hook-and-loop fastener) was prepared, and was allowed to stand still on the female member so that its 25 mm sides were parallel to the flow direction of the engaging layer of the female member. On the resultant, a 0.7 kg roller was laminated once at a speed of 300 mm/minute to perform pressure bonding. After that, the guide was held and pulled in the TD direction of the engaging layer at a speed of 300 mm/minute, and a force required for the male member to be peeled from the female member was measured. AG-20kNG manufactured by Shimadzu Corporation was used as a measuring machine, and a chuck-to-chuck distance was set to 60 mm.

### <Peeling Strength>

An obtained hook-and-loop fastener female member was cut to a size of 5 cm×5 cm. Meanwhile, a 25 mm×13 mm male member having a guide made of a polyethylene film having a thickness of 60 µm (commercially available mechanical hook member for a hook-and-loop fastener) was prepared, and was allowed to stand still on the female member so that its 25 mm sides were parallel to the flow direction of the engaging layer of the female member. On the resultant, a 0.7 kg roller was laminated once at a speed of 300 mm/minute to perform pressure bonding. After that, the guide was chucked on the upper side of a measuring machine and the hook-and-loop fastener was chucked to the lower side of the measuring machine. The guide and the hook-and-loop fastener were pulled (T-peel) in the TD direction of the engaging layer at a speed of 300 mm/minute, and a force required for the male member to be peeled from the female member was measured. AG-20kNG manufactured by Shimadzu Corporation was used as the measuring machine, and a chuck-to-chuck distance was set to 60 mm. This operation was repeated a total of 4 times. The result at the first time was defined as peeling strength (first time) and the result at the fourth time was defined as peeling strength (fourth time).

### <Evaluation of Fluffing>

The non-woven fabric surface of the engaging layer after the evaluation of peeling strength was visually observed and evaluated for fluffing.
⊚: The degree of fluffing after 4 times of repeated peeling is small.
○: The degree of fluffing after 4 times of repeated peeling is large,
but the degree of fluffing after 1 time of repeated peeling is small.
×: The degree of fluffing after 1 time of peeling is large.

### <Measurement of Fiber Diameter>

Through the use of a digital microscope "VHX-1000" manufactured by Keyence Corporation, a non-woven fabric surface was photographed at a magnification of 500, and fiber diameters were measured for N=5 or more with the image analysis software of the digital microscope. The average value of the measured fiber diameters was defined as a fiber diameter. When the fiber diameter increased in a thickness direction (e.g., SMS or SSMMS), the same number of fiber diameters were measured for N=5 or more in each piece of fiber, and the average value of the measured fiber diameters was defined as a fiber diameter. A portion having a locally small thickness due to heat fusion or the like as in spunbond, spunmelt, or the like is not included.

### <Thickness of Non-woven Fabric>

Through the use of a digital microscope "VHX-1000" manufactured by Keyence Corporation, a cross-section of a non-woven fabric was photographed at a magnification of 100, and the thickness of the non-woven fabric was measured with the image analysis software of the digital microscope. In the measurement of the thickness, parallel lines drawn so as to have 10 points of intersection with fiber in an upper portion and a lower portion of the cross-section of the non-woven fabric, respectively, were defined as an upper side and a lower side, respectively, and a length between the upper side and the lower side was defined as the thickness of the non-woven fabric.

### [Example 1]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric produced by a "spunbond method", and the non-woven fabric used for a physical property layer is a non-woven fabric produced by a "spunbond method". The results are shown in Table 1.

### [Example 2]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric of crimpable fiber produced by an "air-through method" and having a core-sheath structure (sheath portion: polyethylene resin, core portion: polyethylene terephthalate resin), and the non-woven fabric used for a physical property layer is a non-woven fabric produced by a "spunbond method". The results are shown in Table 1.

### [Example 3]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric of crimpable fiber produced by an "air-through method" and having a core-sheath structure (sheath portion: polyethylene resin, core portion: polyethylene terephthalate resin), and the non-woven fabric used for a physical property layer is a non-woven fabric produced by a "spunbond method". The results are shown in Table 1.

### [Example 4]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric of crimpable fiber produced by a "spunbond method" and having a core-sheath structure (sheath portion: polyethylene resin, core portion: polyethylene terephthalate resin), and the non-woven fabric used for a physical property layer is a non-woven fabric produced by a "spunbond method". The results are shown in Table 1.

### [Example 5]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric of crimpable fiber produced by an "air-through method" and having a core-sheath structure (sheath portion: polyethylene resin, core portion: polypropylene resin), and the non-woven fabric used for a physical property layer is a non-woven fabric produced by a "spunbond method". The results are shown in Table 1.

### [Example 6]

A raw non-woven fabric and a film shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabric and the side formed an angle of 45°. The non-woven fabric used for an engaging layer is a non-woven fabric produced by a "spunbond method", and the film used for a physical property layer is a polypropylene film having a thickness of 20 µm. The results are shown in Table 1.

### [Example 7]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a straight line having a pitch of 4 mm, and was placed so that the flow direction of the non-woven fabrics and the straight line of the pattern formed an angle of 90°. The non-woven fabric used for an engaging layer is a non-woven fabric of crimpable fiber produced by an "air-through method" and having a core-sheath structure (sheath portion: polyethylene resin, core portion: polyethylene terephthalate resin), and the non-woven fabric used for a physical property layer is a non-woven fabric produced by a "spunbond method". The results are shown in Table 1.

### [Example 8]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a straight line having a pitch of 4 mm, and was placed so that the flow direction of the non-woven fabrics and the straight line of the pattern formed an angle of 90°. The non-woven fabric used for an engaging layer is a non-woven fabric of crimpable fiber produced by an "air-through method" and having a core-sheath structure (sheath portion: polyethylene resin, core portion: polyethylene terephthalate resin), and the non-woven fabric used for a physical property layer is a non-woven fabric produced by a "spunbond method". The results are shown in Table 1.

### [Example 9]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a straight line having a pitch of 4 mm, and was placed so that the flow direction of the non-woven fabrics and the straight line of the pattern formed an angle of 90°. The non-woven fabric used for an engaging layer is a non-woven fabric of crimpable fiber produced by an "air-through method" and having a core-sheath structure (sheath portion: polyethylene resin, core portion: polypropylene resin), and the non-woven fabric used for a physical property layer is a non-woven fabric produced by a "spunbond method" . The results are shown in Table 1.

### [Example 10]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a straight line having a pitch of 4 mm, and was placed so that the flow direction of the non-woven fabrics and the straight line of the pattern formed an angle of 90°. Thenon-woven fabric used for an engaging layer is a non-woven fabric produced by a "spunbond method" , and the non-woven fabric used for a physical property layer is a non-woven fabric produced by a "spunbond method". The results are shown in Table 1.

### [Example 11]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric produced by a "spunbond method", and the non-woven fabric used for a physical property layer is a non-woven fabric produced by a "spunbond method". The results are shown in Table 1.

### [Example 12]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric of crimpable fiber produced by an "air-through method" and having a core-sheath structure (sheath portion: polyethylene resin, core portion: polyethylene terephthalate resin), and the non-woven fabric used for a physical property layer is a non-woven fabric of crimpable fiber produced by a "spunbond method" and having a core-sheath structure (sheath portion: polyethylene resin, core portion: polypropylene resin). The results are shown in Table 1.

### [Comparative Example 1]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric produced by a "spunbond method", and the non-woven fabric used for a physical property layer is a non-woven fabric produced by a "spunbond method".

### [Comparative Example 2]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a square of 10 mm on a side, and was placed so that the flow direction of the non-woven fabrics and the side formed an angle of 45°. The non-woven fabric used for an engaging layer is a non-woven fabric produced by a "spunmelt method", and the non-woven fabric used for a physical property layer is a non-woven fabric produced by a "spunbond method".

### [Comparative Example 3]

Raw non-woven fabrics shown in Table 1 were laminated, and were subjected to embossing treatment with a pattern roll at a treatment temperature of 160°C, a linear pressure of 160 N/mm, and a treatment speed of 10 m/minute to provide a hook-and-loop fastener female member. The pattern used had an embossing portion having a width of 1 mm, was a straight line having a pitch of 4 mm, and was placed so that the flow direction of the non-woven fabrics and the straight line of the pattern formed an angle of 90°. The non-woven fabric used for an engaging layer is a non-woven fabric produced by an "air-through method", and the non-woven fabric used for a physical property layer is a non-woven fabric produced by a "spunbond method". The results are shown in Table 1. Examples 1-3, 6-9 and 12 are not according to the invention. Examples 4,5,10 and 11 are according to the invention.

**Table 1**

| | | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 | Comparative Example 3 |
|---|---|---|---|---|---|---|---|---|---|---|---|---|---|---|---|---|
| Non-woven fabric of engaging layer | Fiber composition | PP | PE/PE T | PE/PE T | PE/PE T | PE/PP | PP | PE/PE T | PE/PE T | PE/PP | PP | PP | PE/PE T | PP | PP | PP |
| | Fiber diameter [µm] | 19.2 | 27.3 | 27.2 | 36.1 | 55.2 | 19.2 | 31.6 | 31.3 | 20.0 | 21.8 | 21.8 | 27.2 | 12.2 | 9.0 | 15.8 |
| | Basis weight of non-woven fabric [g/m²] | 21 | 50 | 40 | 50 | 35 | 21 | 30 | 20 | 18 | 18 | 18 | 40 | 20 | 13 | 17 |
| | Thickness [mm] | 0.70 | 4.5 | 1.17 | 0.83 | 0.49 | 0.70 | 1.12 | 2.19 | 0.83 | 0.19 | 0.19 | 1.17 | 0.51 | 0.13 | 0.14 |
| | Density of non-woven fabric [kg/m³] | 30.0 | 11.1 | 34.2 | 60.4 | 71.0 | 30.0 | 26.8 | 9.1 | 21.6 | 94.3 | 94.3 | 34.2 | 39.1 | 104.0 | 117.5 |
| Physical property layer | Construction | Non-woven fabric | Non-woven fabric | Non-woven fabric | Non-woven fabric | Non-woven fabric | Film | Non-woven fabric | Non-woven fabric | Non-woven fabric | Non-woven fabric | Non-woven fabric | Non-woven fabric | Non-woven fabric | Non-woven fabric | Non-woven fabric |
| | Composition | PP | PP | PP | PP | PP | PP | PP | PP | PP | PP | PP | PE/PP | PP | PP | PP |
| | Fiber diameter [µm] | 17.8 | 17.8 | 17.8 | 17.6 | 17.8 | - | 19.6 | 19.6 | 19.6 | 19.6 | 19.6 | 19.8 | 17.8 | 17.8 | 19.6 |
| | Basis weight of non-woven fabric [g/m²] | 24 | 24 | 24 | 15 | 24 | - | 15 | 15 | 15 | 15 | 15 | 20 | 24 | 24 | 15 |
| | Film thickness [µm] | - | - | - | - | - | 20 | - | - | - | - | - | - | - | - | - |
| Shearing stress [N] | | 10.9 | 25.6 | 25.5 | 13.6 | 12.1 | 11.1 | 31.3 | 30.8 | 33.6 | 18.5 | 9.4 | 25.8 | 6.8 | 0.4 | 1.1 |
| Peeling strength (first time) [N] | | 0.9 | 1.1 | 1.2 | 1.2 | 1.0 | 1.0 | 2.0 | 1.9 | 0.6 | 0.5 | 0.9 | 1.5 | 1.1 | 0.1 | 0.1 |
| Peeling strength (fourth time) [N] | | 1.3 | 0.9 | 1.3 | 0.9 | 0.9 | 1.2 | 1.6 | 1.3 | 0.4 | 0.7 | 1.2 | 1.5 | - | 0.1 | 0.0 |
| Fluffing | | ○ | ○ | ○ | ○ | ○ | ○ | ○ | ○ | ○ | ⊚ | ⊚ | ○ | × (Broken) | ○ | ⊚ |

The hook-and-loop fastener female member of the present invention can be used for any appropriate article in which the effect of the present invention can be effectively utilized. A typical example of such article is a sanitary article. Examples of such sanitary article include a diaper (in particular, a disposable diaper), a supporter, and a mask.

### Reference Signs List

- **100**: hook-and-loop fastener female member
- **10**: engaging layer
- **20**: physical property layer

## Claims

1. A hook-and-loop fastener female member, comprising an engaging layer engageable with a male member, and a physical property layer,
wherein the engaging layer includes a non-woven fabric of fiber,
wherein the non-woven fabric in the engaging layer has a density of from 50 kg/m³ to 100 kg/m³,
wherein the fiber has a diameter of from 15 µm to 60 µm,
wherein the engaging layer is laminated on the physical property layer by embossing using a pattern roll,
wherein a surface of the fiber forming the non-woven fabric included in the engaging layer and a surface of the physical property layer on an engaging layer side contain the same kind of polymer, wherein the polymer comprises polyolefin, and
wherein the hook-and-loop fastener female member has a shearing stress of 8 N to 100 N.

2. The hook-and-loop fastener female member according to claim 1, wherein the fiber comprises a polyolefin fiber, and wherein the polyolefin fiber comprises polypropylene fiber.

3. The hook-and-loop fastener female member according to claim 1, wherein the fiber comprises a composite fiber of two or more kinds of resins, wherein the composite fiber of two or more kinds of resins comprises crimpable fiber having a core-sheath structure, wherein a sheath portion of the core-sheath structure contains a polyethylene resin.

4. The hook-and-loop fastener female member according to claim 3, wherein a core portion of the core-sheath structure contains a polyethylene terephthalate resin.

5. The hook-and-loop fastener female member according to claim 1, wherein the non-woven fabric in the engaging layer has a density of from 70 kg/m³ to 100 kg/m³.

6. The hook-and-loop fastener female member according to claim 1, wherein the fiber has a diameter of from 15 µm to 40 µm, preferably from 20 µm to 40 µm.

7. The hook-and-loop fastener female member according to claim 1, wherein the physical property layer includes a non-woven fabric of fiber, and the fiber has a diameter of 40 µm or less.

8. The hook-and-loop fastener female member according to claim 1, wherein the physical property layer comprises a film, and the film has a thickness of 60 µm or less.

9. The hook-and-loop fastener female member according to claim 1, wherein the hook-and-loop fastener female member has a shearing stress of 10 N or more, preferably 20 N or more.

10. A hook-and-loop fastener, comprising:
the hook-and-loop fastener female member of claim 1; and
a hook-and-loop fastener male member configured to engage with the hook-and-loop fastener female member.

11. A sanitary article, comprising the hook-and-loop fastener female member of claim 1.

## Patentansprüche

1. Aufnahmeelement eines Klettverschlusses, umfassend eine Eingriffsschicht, die mit einem Steckelement ineinandergreifen kann, und eine Schicht mit physikalischen Eigenschaften,
wobei die Eingriffsschicht einen Faservliesstoff enthält,
wobei der Vliesstoff in der Eingriffsschicht eine Dichte von 50 kg/m³ bis 100 kg/m³ aufweist,
wobei die Faser einen Durchmesser von 15 µm bis 60 µm aufweist,
wobei die Eingriffsschicht durch Prägen unter Verwendung einer Musterrolle auf die Schicht mit den physikalischen Eigenschaften laminiert ist,
wobei eine Oberfläche der Faser, die den in der Eingriffsschicht enthaltenen Vliesstoff bildet, und eine Oberfläche der Schicht mit den physikalischen Eigenschaften auf einer Seite der Eingriffsschicht die gleiche Art von Polymer enthalten, wobei das Polymer Polyolefin umfasst, und
wobei das Aufnahmeelement eines Klettverschlusses eine Scherspannung von 8 N bis 100 N aufweist.

2. Aufnahmeelement eines Klettverschlusses nach Anspruch 1, wobei die Faser eine Polyolefinfaser umfasst und wobei die Polyolefinfaser eine Polypropylenfaser umfasst.

3. Aufnahmeelement eines Klettverschlusses nach Anspruch 1, wobei die Faser eine Verbundfaser aus zwei oder mehr Arten von Harzen umfasst, wobei die Verbundfaser aus zwei oder mehr Arten von Harzen eine kräuselbare Faser mit einer Kern-Mantel-Struktur umfasst, wobei ein Mantelabschnitt der Kern-Mantel-Struktur ein Polyethylenharz enthält.

4. Aufnahmeelement eines Klettverschlusses nach Anspruch 3, wobei ein Kernabschnitt der Kern-Mantel-Struktur ein Polyethylenterephthalatharz enthält.

5. Aufnahmeelement eines Klettverschlusses nach Anspruch 1, wobei der Vliesstoff in der Eingriffsschicht eine Dichte von 70 kg/m³ bis 100 kg/m³ aufweist.

6. Aufnahmeelement eines Klettverschlusses nach Anspruch 1, wobei die Faser einen Durchmesser von 15 µm bis 40 µm, vorzugsweise von 20 µm bis 40 µm, aufweist.

7. Aufnahmeelement eines Klettverschlusses nach Anspruch 1, wobei die Schicht mit den physikalischen Eigenschaften einen Faservliesstoff enthält und die Faser einen Durchmesser von 40 µm oder weniger aufweist.

8. Aufnahmeelement eines Klettverschlusses nach Anspruch 1, wobei die Schicht mit den physikalischen Eigenschaften einen Film umfasst und der Film eine Dicke von 60 µm oder weniger aufweist.

9. Aufnahmeelement eines Klettverschlusses nach Anspruch 1, wobei das Aufnahmeelement eines Klettverschlusses eine Scherspannung von 10 N oder mehr, vorzugsweise 20 N oder mehr, aufweist.

10. Klettverschluss, umfassend:
das Aufnahmeelement eines Klettverschlusses nach Anspruch 1; und
ein Steckelement eines Klettverschlusses, das konfiguriert ist, um mit dem Aufnahmeelement des Klettverschlusses ineinander zu greifen.

11. Hygieneartikel, umfassend das Aufnahmeelement eines Klettverschlusses nach Anspruch 1.

## Revendications

1. Élément femelle de fixation par crochets et boucles, comprenant une couche engageante s'engageant avec un élément mâle, et une couche de propriété physique,
dans lequel la couche engageante comprend un tissu non tissé de fibre,
dans lequel le tissu non tissé dans la couche engageante présente une densité allant de 50 kg/m³ à 100 kg/m³,
dans lequel la fibre présente un diamètre allant de 15 µm à 60 µm,
dans lequel la couche engageante est laminée sur la couche de propriété physique par gaufrage en utilisant un rouleau à motif,
dans lequel une surface de la fibre formant le tissu non tissé compris dans la couche engageante et une surface de la couche de propriété physique sur le côté couche engageante contiennent le même type de polymère, dans lequel le polymère comprend de la polyoléfine, et
dans lequel l'élément femelle de fixation à boucles et boucles présente une contrainte de cisaillement de 8 N à 100 N.

2. Élément femelle de fixation par crochets et boucles selon la revendication 1, dans lequel la fibre comprend une fibre de polyoléfine et dans lequel la fibre de polyoléfine comprend une fibre de polypropylène.

3. Élément femelle de fixation par crochets et boucles selon la revendication 1, dans lequel la fibre comprend une fibre composite de deux types ou plus de résines, dans lequel la fibre composite de deux types ou plus, de résines, comprend une fibre crêpable présentant une structure noyau-gaine, dans lequel la partie formant gaine de la structure noyau-gaine contient une résine de polyéthylène.

4. Élément femelle de fixation par crochets et boucles selon la revendication 3, dans lequel la partie formant noyau de la structure noyau-gaine contient une résine de poly(téréphtalate d'éthylène).

5. Élément femelle de fixation par crochets et boucles selon la revendication 1, dans lequel le tissu non tissé dans la couche engageante présente une densité allant de 70 kg/m³ à 100 kg/m³.

6. Élément femelle de fixation par crochets et boucles selon la revendication 1, dans lequel la fibre présente un diamètre allant de 15 µm à 40 µm, allant de préférence de 20 µm à 40 µm.

7. Élément femelle de fixation par crochets et boucles selon la revendication 1, dans lequel la couche de propriété physique comprend un tissu non tissé de fibre, et la fibre présente un diamètre de 40 µm ou moins.

8. Élément femelle de fixation par crochets et boucles selon la revendication 1, dans lequel la couche de propriété physique comprend une pellicule, et la pellicule présente une épaisseur de 60 µm ou moins.

9. Élément femelle de fixation par crochets et boucles selon la revendication 1, dans lequel l'élément femelle de fixation par crochets et boucles présente une contrainte de cisaillement de 10 N ou plus, de préférence de 20 N ou plus.

10. Fixation par crochets et boucles, comprenant:
l'élément femelle de fixation par crochets et boucles selon la revendication 1; et
un élément mâle de fixation par crochets et boucles configuré pour se prendre avec l'élément femelle de fixation par crochets et boucles.

11. Article sanitaire, comprenant l'élément femelle de fixation par crochets et boucles selon la revendication 1.
